# EUROPEAN PATENT APPLICATION

(11) **EP 4 442 242 A1**
(43) Date of publication of application: **09.10.2024**
(21) Application number: 22898469.6
(22) Date of filing: 16.11.2022
(51) Int. Cl.: A61K 8/34, A61K 8/49, A61Q 19/00, A61Q 19/02

(54) **COSMETIC**

(30) Priority: 29.11.2021 JP 2021193419
(71) Applicant: Shiseido Company, Ltd., Chuo-ku Tokyo 104-0061 (JP)
(72) Inventor: KOMAI Ryota, Tokyo 104-0061 (JP); OKUYAMA Yusuke, Tokyo 104-0061 (JP)
(74) Representative: Ter Meer Steinmeister & Partner
(86) International application number: PCT/JP2022/042469
(87) International publication number: WO 2023/095685

(57) **Abstract**

The present invention provides a cosmetic that achieves both a high whitening effect and a low feeling of irritation. The cosmetic according to the present invention includes phenylethylresorcinol and an organic acid or a salt thereof represented by the following general formula (I): 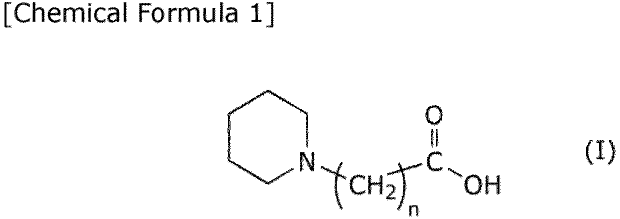 wherein n represents an integer of 2 to 5. The cosmetic can further include vitamin C as necessary.

## Description

### TECHNICAL FIELD

The present invention relates to a cosmetic capable of obtaining a sufficient whitening effect while suppressing a feeling of irritation.

### BACKGROUND ART

Pigmentation such as pigmented spots and freckles of skin is caused by generation of melanin in epidermal pigmented cells triggered by abnormalities of hormones, stimulation of ultraviolet rays, and the like, and deposition of the melanin on the skin. In order to prevent such pigmentation, it is known to use substances that suppress melanin generation, such as a resorcinol derivative, an ascorbic acid derivative, and a placenta extract. Among these substances, a resorcinol derivative, particularly phenylethylresorcinol, is useful as a skin whitening agent because it has an excellent effect. However, when the phenylethylresorcinol is applied to the skin, there is a feeling of irritation to skin, and in a case where the phenylethylresorcinol is blended in a cosmetic, it is necessary to suppress the blending amount to a relatively low concentration so that the irritation becomes weak. As a result, improvement of the whitening effect has also been limited.

### CITATION LIST

### PATENT LITERATURE

Patent Literature 1: JP 4865251 B2

### SUMMARY OF THE INVENTION

In order to solve the above problems, it has been desired to develop a technique capable of suppressing a feeling of irritation to the skin even when the phenylethylresorcinol is blended in a cosmetic.

According to the present invention, the following invention is provided.
[1] A cosmetic including:
   (A) phenylethylresorcinol; and
   (B) an organic acid or a salt thereof represented by the following general formula (I): 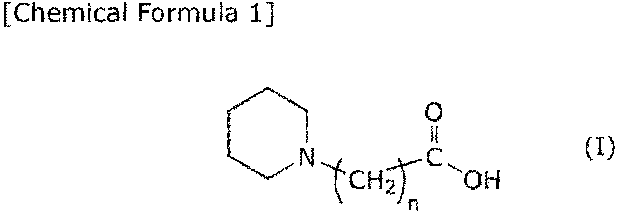 wherein, n represents an integer of 2 to 5.
[2] The cosmetic according to [1], wherein a blending amount of phenylethylresorcinol is 0.01 to 1 mass% based on a total mass of the cosmetic.
[3] The cosmetic according to [1] or [2], wherein a blending amount of the organic acid or the salt thereof is 0.005 to 5 mass% based on the total mass of the cosmetic.
[4] The cosmetic according to any one of [1] to [3], wherein a mass ratio of the blending amount of phenylethylresorcinol to the blending amount of the organic acid or the salt thereof is 0.025 to 10. [5] The cosmetic according to any one of [1] to [4], wherein the organic acid is 1-piperidinepropionic acid.
[6] The cosmetic according to any one of [1] to [5], further including vitamin C or a derivative thereof.
[7] The cosmetic according to [6], wherein a blending amount of vitamin C or the derivative thereof is 0.005 to 1 mass% based on the total mass of the cosmetic.
[8] The cosmetic according to [6] or [7], wherein a mass ratio of the blending amount of vitamin C or the derivative thereof to the blending amount of organic acid is 1/300 to 1.

According to the present invention, there is provided a cosmetic with a large blending amount of phenylethylresorcinol having suppressed skin irritation and a high whitening effect.

### BEST MODE FOR CARRYING OUT THE INVENTION

Hereinafter, embodiments of the present invention will be described in detail.

The cosmetic according to the present invention includes (A) phenylethylresorcinol and (B) a specific organic acid as essential components.

### [Phenylethylresorcinol]

The cosmetic according to the present invention includes phenylethylresorcinol, and phenylethylresorcinol is used in fields of cosmetic products, pharmaceuticals, quasi-drugs, and the like, and can be arbitrarily selected from among them also in the present invention.

There are several isomers of phenylethylresorcinol, such as 4-(1-phenylethyl)resorcinol, 4-(2-phenylethyl)resorcinol, and 3-(1-phenylethyl)resorcinol. Any of these can be used as the cosmetic according to the present invention, but 4-(1-phenylethyl)resorcinol among these can be easily accessed, and a high effect can be obtained, which is preferable.

The blending amount of phenylethylresorcinol is not particularly limited as long as it is an amount effective for exhibiting the effect of the component, but is usually preferably 0.01 to 1 mass% and more preferably 0.05 to 0.7 mass% based on the total mass of the cosmetic. In the present invention, when a specific organic acid to be described later is used in combination, a content of phenylethylresorcinol in such a range can be adopted, and a sufficient whitening effect can be obtained with irritation suppressed.

### [Organic acid or salt thereof]

The cosmetic according to the present invention includes a specific organic acid or a salt thereof. This organic acid is represented by the following general formula (I): 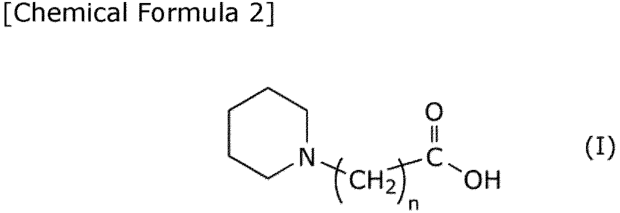 wherein n represents an integer of 2 to 5.

The organic acid is, depending on the number of n, 1-piperidinepropionic acid (n = 2), 4-(1-piperidinyl)butanoic acid (n = 3), 5-(1-piperidinyl)pentanoic acid (n = 4), or 6-(1-piperidinyl)hexanoic acid (n = 5), and among these, 1-piperidinepropionic acid is particularly preferable because it not only suppresses the irritation caused by phenylethylresorcinol, but also improves skin quality of the skin in terms of being less likely to be rough, and has an effect of suppressing pigmentation.

The salt of such an organic acid is not particularly limited, and examples of the inorganic salt include hydrochloride, sulfate, phosphate, hydrobromide, sodium salt, potassium salt, magnesium salt, calcium salt, and ammonium salt. Examples of the organic salt include acetate, lactate, maleate, fumarate, tartrate, citrate, methanesulfonate, p-toluenesulfonate, triethanolamine, diethanolamine, and amino acid salts.

The blending amount of the organic acid or the salt thereof is not particularly limited, but is preferably 0.005 to 5 mass%, and more preferably 0.01 to 4 mass%, based on the total mass of the cosmetic. When the blending amount of the organic acid or the salt thereof is within this range, it is possible to provide a cosmetic that can suppress a feeling of irritation, has an excellent moisturizing feeling, has less sticky feeling, and has an excellent usability.

In addition, in the present invention, in order to optimally maintain a balance between a whitening effect by phenylethylresorcinol and a suppressing effect on the feeling of irritation by the organic acid or the salt thereof, a mass ratio (a/b) of a blending amount (a) of phenylethylresorcinol to a blending amount (b) of the organic acid or the salt thereof is preferably 0.025 to 10, and more preferably 0.1 to 5.

### [Other components]

The cosmetic according to the present invention includes the phenylethylresorcinol described above and the organic acid or the salt thereof as essential components, but may include other components as necessary.

Among such components, the vitamin C or the derivative thereof can be mentioned as a component which is preferably formulated in the cosmetic product according to the present invention. The vitamin C is known as a nutrient and corresponds to L-ascorbic acid as a compound. The vitamin C or the derivative thereof are commonly used in cosmetic products. The vitamin C or the derivative thereof is known to exhibit a whitening effect and the like, and in the present invention, the vitamin C or the derivative thereof exhibits an effect of compensating the suppressing effect on the feeling of irritation by the organic acid or the salt thereof.

The vitamin C is water-soluble, and water-soluble, oil-soluble, and amphiphilic derivatives thereof are known, but water-soluble derivatives are preferably used. Specific examples thereof include 2-O-ethylascorbic acid, 3-O-ethylascorbic acid, Na ascorbylphosphate, Mg ascorbylphosphate, ascorbylglucoside, glycerylascorbate, 3-glycerylascorbate, bis-glycerylascorbate, and L-ascorbylpalmitate.

The blending amount of vitamin C or the derivative thereof is not particularly limited, but is preferably 0.005 to 1 mass%, and more preferably 0.008 to 0.2 mass%, based on the total mass of the cosmetic. When the blending amount of vitamin C or the derivative thereof is within this range, it is possible to provide a cosmetic that is excellent in whitening effect and usability with less irritation.

In the present invention, in order to optimally maintain the balance between the whitening effect and the suppressing effect on the feeling of irritation, a mass ratio (c/b) of a blending amount (c) of vitamin C or the derivative thereof to the blending amount (b) of the organic acid or the salt thereof is preferably 1/300 to 1, and more preferably 1/200 to 1/3.

In addition, the cosmetic product according to the present invention is appropriately blended as necessary with components usually used in cosmetic products, pharmaceuticals, and the like, for example, water, oil components, surfactants, powders, coloring materials, alcohols, thickeners, chelating agents, silicones, antioxidants, ultraviolet absorbers, moisturizers, fragrances, various medicinal ingredients, preservatives, pH adjusting agents, neutralizing agents, and the like.

In the cosmetic according to the present invention, it is preferable to blend a large amount of water in order to form a cosmetic liquid having a whitening effect or an emulsion in particular. As the water, water used for cosmetic products, quasi-drugs, and the like can be used, and for example, purified water, ion-exchanged water, tap water, and the like can be used. The blending amount of water varies depending on the blending amount of other components, but is generally preferably 20 to 80 mass% and more preferably 30 to 70 mass% based on the total mass of the cosmetic.

The cosmetic according to the invention can include a polyhydric alcohol. Such a polyhydric alcohol can be arbitrarily selected as long as the effect of the present invention is not impaired. Specifically, the polyhydric alcohol is preferably one kind or two or more kinds selected from a group consisting of glycerin, diglycerin, polyglycerin, 1,3-butyleneglycol, ethyleneglycol, propyleneglycol, dipropyleneglycol, polyethyleneglycol, polypropyleneglycol, polyethyleneglycol/polypropyleneglycol, trimethylolethane, trimethylolpropane, erythritol, pentaerythritol, sorbitan, glucose, sorbitol, maltitol, sucrose, raffinose, hexyleneglycol, 1,2-pentanediol, and trehalose.

The polyhydric alcohol exerts a moisturizing function and a thermal sensation imparting function, and the blending amount thereof is adjusted depending on purposes. In general, the blending amount of polyhydric alcohol is preferably 1 to 30 mass%, and more preferably 5 to 20 mass%, based on the total mass of the cosmetic.

In the present invention, the cosmetic according to the present invention can include a higher alcohol, and the higher alcohol is not particularly limited as long as it can be used in fields of cosmetic products, pharmaceuticals, quasi-drugs, and the like. Specific examples thereof include lauryl alcohol, myristyl alcohol, cetyl alcohol, stearyl alcohol, and behenyl alcohol. In addition, mixtures thereof, for example, cetearyl alcohol (cetostearyl alcohol) which is a mixture of stearyl alcohol and cetyl alcohol, and the like are generally known, and can also be used.

In a case of blending the higher alcohol, one kind or two or more kinds described above can be used. In the present invention, it is preferable to use a mixture of two or more kinds of aliphatic alcohols, and it is more preferable to use such a combination that a melting point of the mixture is 60°C or higher. If the melting point is lower than 60°C, temperature stability of the system may be deteriorated depending on formulations. In the present invention, for example, a combination of stearyl alcohol and behenyl alcohol is preferable.

The blending amount of higher alcohol is preferably 0.1 to 10 mass%, and more preferably 0.5 to 5 mass% with respect to the total mass of the cosmetic.

The cosmetic according to the present invention can include a surfactant. As the surfactant, various surfactants such as nonionic surfactants, cationic surfactants, anionic surfactants, amphoteric surfactants, and silicone surfactants are known, and any surfactant selected from them can be used. The blending amount of surfactant is preferably 0.1 to 20 mass%, and more preferably 0.3 to 5 mass% with respect to the total amount of the oil-in-water type emulsified cosmetic.

The cosmetic according to the present invention can include any oil component depending on purposes. The oil component that can be used in the present invention is not particularly limited, and can be selected from those used in cosmetic products as long as the stability is not impaired. Examples of preferable oil components include polar oils such as hydrocarbon oils and ester oils, silicone oils, and liquid oil-and-fat.

Specifically,
examples of the hydrocarbon oil include liquid paraffin, squalane, squalene, paraffin, isoparaffin, ceresin, isohexadecane, and isododecane;
examples of polar oils such as ester oils include pentaerythrityltetraethylhexanoate, cetylethylhexanoate, jojoba oil, di(phytosteryl/octyldodecyl)lauroylglutamate, triisostearate, glyceryldiisostearate, triethylhexanoin, phytosteryl/behenyl dimer dilinoleate, phytosteryl/isostearyl/cetyl/stearyl/isostearyl/cetyl/stearyl/behenyl dimer dilinoleate, isopropylpalmitate, macadamia nut fatty acid phytosteryl, pentaerythrityl tetrabehenate/benzoate/ethylhexanoate, ethylhexylpalmitate, myristylmyristate, isopropylmyristate, tripropyleneglycoldipivalate, diisopropylsebacate, and isodecylneopentanoate;
examples of the silicone oil include chain silicones such as dimethylpolysiloxane, methylphenylpolysiloxane, and methylhydrogenpolysiloxane; cyclic silicones such as octamethylcyclotetrasiloxane, decamethylcyclopentasiloxane, and dodecamethylcyclohexasiloxane; silicone resins forming a three-dimensional network structure; and silicone rubbers; and
examples of the liquid oil-and-fat include linseed oil, camellia oil, macadamia nut oil, corn oil, mink oil, olive oil, avocado oil, sasanqua oil, castor oil, safflower oil, rapeseed oil, soybean oil, peanut oil, triglycerin, glycerintrioctanoate, glycerintrioctanoate, and glycerintriisopalmitate. In a case where these oil components are included, the oil-in-water type cosmetic is preferable.

The blending amount of oil component is not particularly limited, but is preferably 5 to 40 mass% with respect to the total amount of the oil-in-water type emulsified cosmetic.

The cosmetic according to the invention can be formulated with further components depending on purposes. Examples of such components include amino acids, bactericides, anti-inflammatory agents, astringents, animal and plant extracts, vitamins other than vitamin C and derivatives thereof, chelating agents, dyes, antioxidants, preservatives, and perfumes.

### [Cosmetic]

The cosmetic according to the present invention includes the essential components described above, but can be used in all dosage forms that can be taken by general cosmetics. For example, specifically, it can be a dosage form such as a serum, an emulsion, a lotion, a cream, or an aerosol. In any of the dosage forms, it is possible to provide a cosmetic having both less feeling of irritation and excellent usability while achieving a whitening effect.

### Examples

### [Examples 1 to 11, and Comparative Examples 1 and 2]

Cosmetics of the examples and the comparative examples were prepared with the formulations shown in Table 1.

Furthermore, these cosmetics were evaluated for feeling of irritation and moisturizing feeling during use according to the following criteria.

### [Evaluation method]

An actual use test by 20 specialized panelists was performed using the cosmetic of each example. Test items are lack of feeling of irritation and moisturizing feeling. Each expert panelist evaluated each test item based on the following evaluation point criteria, and ranked the cosmetic by the sum of the evaluation points.

Evaluation point criteria (feeling of irritation and moisturizing feeling):
5 points: Excellent.
4 points: Good.
3 points: Ordinary.
2 points: Poor.
1 point: Very poor.

Evaluation rank of feeling of irritation
A+: 90 or more points in total
A: 80 or more points in total
B: 60 points or more and less than 80 points in total
C: 40 points or more and less than 60 points in total
D: less than 40 points in total

Evaluation rank of moisturizing feeling
A: 80 or more points in total
B: 60 points or more and less than 80 points in total
C: 40 points or more and less than 60 points in total
D: less than 40 points in total

### [Table 1]

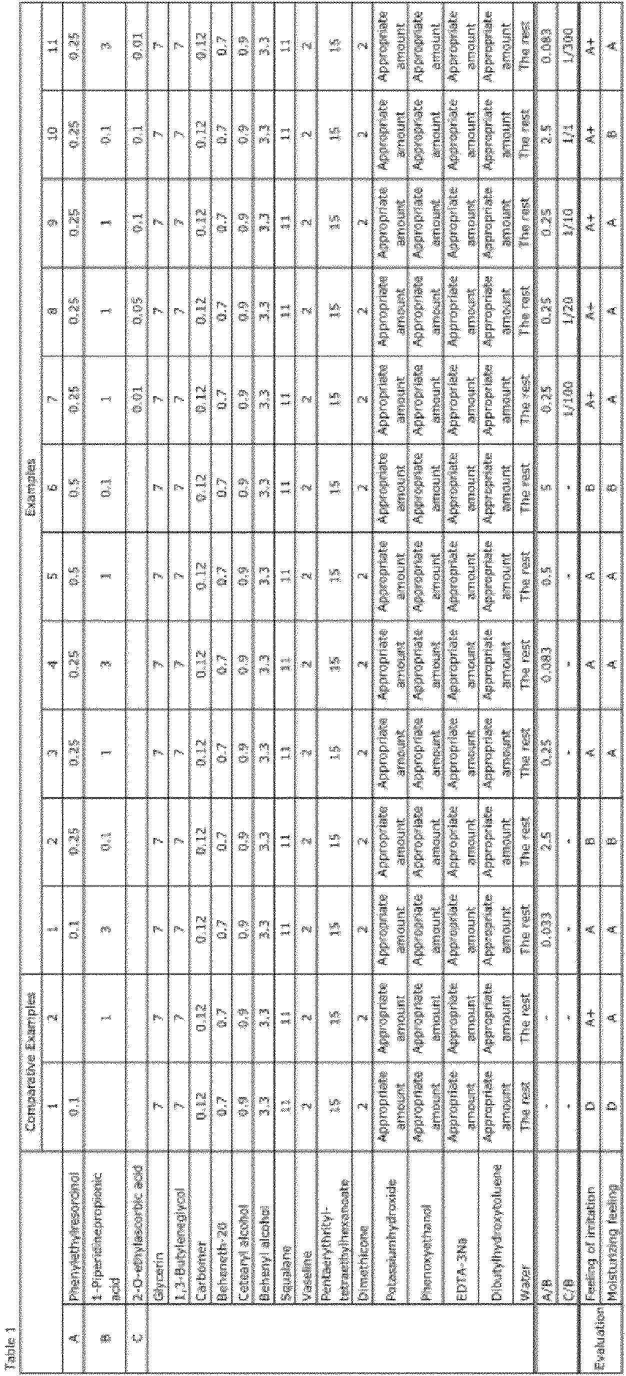

From the results of Comparative Example 1, it is found that the feeling of irritation and the moisturizing feeling tend to be considerably deteriorated by blending the phenylethylresorcinol. However, in the examples, even when the blending amount of phenylethylresorcinol is increased to about 5 times that in Comparative Example 1, the blending amount is improved to a level comparable to that in Comparative Example 2 in which phenylethylresorcinol is not blended. On the other hand, in Comparative Example 2, since phenylethylresorcinol is not included, irritation is small, but it is obvious that the whitening effect, which is one of the objects of the present invention, cannot be obtained. From the above results, it was found that in the cosmetic according to the present invention, since phenylethylresorcinol can be blended at a high blending ratio, an excellent whitening effect can be achieved, and at the same time, an excellent moisturizing feeling can be obtained with less irritation.

## Claims

1. A cosmetic comprising:
(A) phenylethylresorcinol, and
(B) an organic acid or a salt thereof represented by a following general formula (I): 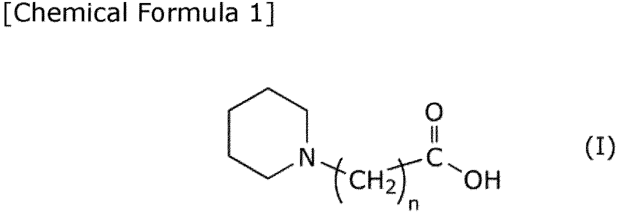 wherein n represents an integer of 2 to 5.

2. The cosmetic according to claim 1, wherein a blending amount of the phenylethylresorcinol is 0.01 to 1 mass% based on a total mass of the cosmetic.

3. The cosmetic according to claim 1 or 2, wherein a blending amount of the organic acid or the salt thereof is 0.005 to 5 mass% based on the total mass of the cosmetic.

4. The cosmetic according to claim 1 or 2, wherein a mass ratio of the blending amount of phenylethylresorcinol to a blending amount of the organic acid or the salt thereof is 0.025 to 10.

5. The cosmetic according to claim 1 or 2, wherein the organic acid is 1-piperidinepropionic acid.

6. The cosmetic according to claim 1 or 2, further comprising vitamin C or a derivative thereof.

7. The cosmetic according to claim 6, wherein a blending amount of vitamin C or the derivative thereof is 0.005 to 1 mass% based on the total mass of the cosmetic.

8. The cosmetic according to claim 6 or 7, wherein a mass ratio of the blending amount of vitamin C or the derivative thereof to the blending amount of organic acid is 1/300 to 1.
